(19) Europäisches Patentamt
European Patent Office
Office européen des brevets

(11) EP 3 990 173 B1

(12) **FASCICULE DE BREVET EUROPEEN**

(45) Date de publication et mention de la délivrance du brevet:
**03.09.2025 Bulletin 2025/36**

(21) Numéro de dépôt: **20731886.6**

(22) Date de dépôt: **16.06.2020**

(51) Classification Internationale des Brevets (IPC):
***B01D 53/86*** (2006.01) ***B01J 37/03*** (2006.01)
***B01J 27/04*** (2006.01) ***B01J 37/10*** (2006.01)
***C07C 1/02*** (2006.01) ***B01J 35/33*** (2024.01)
***B01J 27/043*** (2006.01) ***B01J 35/39*** (2024.01)
***B01J 35/64*** (2024.01) ***B01J 35/70*** (2024.01)

(52) Classification Coopérative des Brevets (CPC):
(C-Sets disponibles)
**B01J 35/39; B01D 53/8671; B01J 27/04; B01J 27/043; B01J 35/33; B01J 35/643; B01J 35/70; B01J 37/031; B01J 37/10; C07C 1/02;**
B01D 2255/802; B01D 2257/504; B01J 2235/00; B01J 2235/15; B01J 2235/30; (Cont.)

(86) Numéro de dépôt international:
**PCT/EP2020/066568**

(87) Numéro de publication internationale:
**WO 2020/260064 (30.12.2020 Gazette 2020/53)**

(54) **PROCEDE DE REDUCTION PHOTOCATALYTIQUE DU CO2 METTANT EN OEUVRE UN PHOTOCATALYSEUR DE TYPE SULFURE METALLIQUE CRISTALLISE MICROPOREUX**

VERFAHREN ZUR KATALYTISCHEN REDUKTION VON CO2 UNTER VERWENDUNG EINES METALLSULFID-BESTEHENDEN PHOTOKATALYSATORS

METHOD FOR THE PHOTOCATALYTIC REDUCTION OF CO2 INVOLVING A MICROPOROUS CRYSTALIZED METALLIC SULFIDE PHOTOCATALYST

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.06.2019 FR 1907102**

(43) Date de publication de la demande:
**04.05.2022 Bulletin 2022/18**

(73) Titulaire: **IFP Energies nouvelles**
**92852 Rueil-Malmaison Cedex (FR)**

(72) Inventeurs:
- **FECANT, Antoine**
  **92852 RUEIL-MALMAISON CEDEX (FR)**
- **MARTINEZ FRANCO, Raquel**
  **92852 RUEIL-MALMAISON CEDEX (FR)**
- **PIRNGRUBER, Gerhard**
  **92852 RUEIL-MALMAISON CEDEX (FR)**
- **LEFLAIVE, Philibert**
  **92852 RUEIL-MALMAISON CEDEX (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
**CN-A- 106 006 717** **CN-A- 109 046 385**
**CN-A- 109 225 273** **CN-A- 109 331 883**
**US-A1- 2013 252 798**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

(52) Classification Coopérative des Brevets (CPC):
(Cont.)C07C 2521/06; C07C 2523/14;
C07C 2527/04; C07C 2527/043

C-Sets
**C07C 1/02, C07C 9/04**

## Description

### Domaine technique

Le domaine de l'invention est celui de la réduction photocatalytique du dioxyde de carbone ($CO_2$) sous irradiation par l'emploi d'un photocatalyseur.

### Etat de la technique

Les combustibles fossiles, comme le charbon, le pétrole et le gaz naturel, sont les principales sources d'énergie conventionnelles dans le monde en raison de leur disponibilité, de leur stabilité et de leur densité d'énergie élevée. Cependant la combustion produit des émissions de dioxyde de carbone qui sont considérées comme la principale cause du réchauffement climatique. Ainsi, il existe un besoin croissant pour atténuer les émissions de $CO_2$, soit en le captant, soit en le transformant.

Bien que la capture et séquestration « passives » du carbone (CSC) soient généralement considérées comme un procédé efficace pour réduire les émissions de $CO_2$, d'autres stratégies doivent être envisagées, notamment des stratégies « actives » de conversion du $CO_2$ en produits ayant une valeur économique, tels que les carburants et produits chimiques industriels.

De telles stratégies « actives » se basent sur la réduction du dioxyde de carbone en produits valorisables. La réduction du dioxyde de carbone peut être réalisée par voie biologique, thermique, électrochimique ou encore photocatalytique. Parmi ces options, la réduction photocatalytique du $CO_2$ gagne une attention accrue car elle peut potentiellement consommer des formes alternatives d'énergie, par exemple en exploitant l'énergie solaire, qui est abondante, bon marché, et écologiquement propre et sûre.

La réduction photocatalytique du dioxyde de carbone permet d'obtenir des molécules carbonées en $C_1$ ou plus, telles que le monoxyde de carbone (CO), le méthane, le méthanol, l'éthanol, le formaldéhyde, l'acide formique ou encore d'autres molécules telles que les acides carboxyliques, les aldéhydes, les cétones ou différents alcools. Ces molécules peuvent trouver une utilité énergétique directement, telles que le méthanol, l'éthanol, l'acide formique ou encore le méthane et tous les hydrocarbures en $C_1^+$. Le monoxyde de carbone (CO) peut également être valorisé énergétiquement en mélange avec du dihydrogène pour la formation de carburants par synthèse Fischer-Tropsch. Les molécules d'acides carboxyliques, d'aldéhydes, de cétones ou de différents alcools quant à elles peuvent trouver des applications dans les procédés de chimie ou de pétrochimie. Toutes ces molécules présentent donc un grand intérêt d'un point de vue industriel.

La photocatalyse repose sur le principe d'activation d'un semi-conducteur ou d'un ensemble de semi-conducteurs tel qu'un photocatalyseur, à l'aide de l'énergie apportée par l'irradiation. La photocatalyse peut être définie comme l'absorption d'un photon, dont l'énergie est supérieure à la largeur de bande interdite ou "bandgap" selon la terminologie anglo-saxonne entre la bande de valence et la bande de conduction, qui induit la formation d'une paire électron-trou dans le cas d'un semi-conducteur. On a donc l'excitation d'un électron au niveau de la bande de conduction et la formation d'un trou sur la bande de valence. Cette paire électron-trou va permettre la formation de radicaux libres qui vont soit réagir avec des composés présents dans le milieu, afin d'initier des réactions d'oxydo-réduction, ou alors se recombiner suivant divers mécanismes. Un semi-conducteur est caractérisé par sa bande interdite ou « bandgap », i.e. la différence d'énergie entre sa bande de conduction et sa bande de valence qui lui est propre. Tout photon d'énergie supérieure à sa bande interdite peut être absorbé par le semi-conducteur. Tout photon d'énergie inférieure à sa bande interdite ne peut pas être absorbé par le semi-conducteur.

Des procédés de réduction photocatalytique du dioxyde de carbone en présence d'un composé sacrificiel sont connus dans l'état de l'art.

Halmann et al. (Solar Energy, 31, 4, 429-431, 1983) ont évalué les performances de trois semi-conducteurs ($TiO_2$, $SrTiO_3$ et $CaTiO_3$) pour la réduction photocatalytique de $CO_2$ en milieu aqueux. Ils constatent la production de formaldéhyde, d'acide formique et de méthanol.

Anpo et al. (J. Phys. Chem. B, 101, p. 2632-2636, 1997) ont étudié la réduction photocatalytique du $CO_2$ avec de la vapeur d'eau sur des photocatalyseurs à base de $TiO_2$ ancrés dans des micropores de zéolithes. Ceux-ci présentèrent une très haute sélectivité en méthanol gazeux.

Mori et al. (RSC Adv. 2012, 2 (8), 3165-3172) ont mis en évidence l'amélioration de l'activité photocatalytique de matériaux cristallisés microporeux ou mésoporeux à base d'oxyde de titane par rapport à un dioxyde de titane bulk pour la réduction du $CO_2$.

Ainsi, si la mise en œuvre de photocatalyseurs cristallisés microporeux est connue de l'état de l'art, lesdits photocatalyseurs présentent souvent des largeurs de bande interdite élevées (>3eV), ne permettant ainsi de valoriser qu'une part minime des photons du spectre solaire.

Il est connu de l'art antérieur de mettre en œuvre des matériaux de type sulfures métalliques non microporeux (« bulk » selon la terminologie anglo-saxonne) en tant que photocatalyseur (O. Stroyuk et al, Chem. Soc. Rev., 2108, 47, p.

5354). Ce type de matériaux a l'avantage de présenter des largeurs de bande interdite plus faibles (<3 eV) que la plupart des oxydes métalliques, tel que le $TiO_2$ par exemple.

Le document US 2013/252798 divulgue un photocatalyseur sulfure métallique utilisé dans un procédé de réduction du CO2 en CH3OH.

Le document CN109331883 divulgue un photocatalyseur organométallique CdS utilisé pour la réduction du CO2.

Le document CN109225273 divulgue un composite CuS/WS2 comme photocatalyseur utilisé pour la réduction du CO2.

Le document CN109046385 divulgue des photocatalyseurs à base de sulfure de zinc ZnS utilisé pour la réduction du CO2.

Ces trois derniers documents divulguent tous des matériaux à base de soufre utilisés comme photocatalyseurs pour la réduction du CO2 mais aucun de ces documents ne suggèrent de modifier la structure des photocatalyseurs afin de les rendre plus performants dans le procédé de photocatalyse.

Le document CN106006717 divulgue des semiconducteurs à base de sulfure de zinc ZnS sous forme d'aérogel pouvant être utilisés comme photocatalyseurs sans mentionner leur utilisation pour la réduction du CO2.

**Objets de l'invention**

L'objet de l'invention est de proposer une voie nouvelle, durable et plus performante de production de molécules carbonées valorisables par conversion photocatalytique du dioxyde de carbone mettant en oeuvre un photocatalyseur à base de sulfure métallique cristallisé microporeux. Le procédé de réduction photocatalytique de $CO_2$ selon l'invention permet d'atteindre des performances améliorées par rapport aux mises en oeuvre connues pour cette réaction. Les procédés de réduction photocatalytique selon l'art antérieur diffèrent de l'invention par le fait que les semi-conducteurs cristallisés microporeux présentent des largeurs de bande interdite élevées (>3 eV) ne permettant pas aux matériaux d'absorber une quantité importante de photons issus de la partie visible du spectre solaire. En effet, les procédés de réduction photocatalytique selon l'art antérieur utilisent des photocatalyseurs à base de sulfure métallique ne présentant pas de microporosité. Sans être lié à aucune théorie, la présence de microporosité dans le photocatalyseur permet un temps de parcours faible des charges réactives électrons $e^-$et trous $h^+$ et ainsi des taux de recombinaison plus faibles.

Plus particulièrement, l'invention décrit un procédé de réduction photocatalytique du dioxyde de carbone effectué en phase liquide et/ou en phase gazeuse, ledit procédé comprenant les étapes suivantes :

a) on met en contact une charge contenant le dioxyde de carbone et au moins un composé sacrificiel avec un photocatalyseur comprenant au moins un semi-conducteur à base de sulfure métallique cristallisé microporeux ;
b) on irradie le photocatalyseur par au moins une source d'irradiation produisant au moins une longueur d'onde inférieure à la largeur de bande interdite dudit photocatalyseur, ladite étape b) étant réalisée à une température comprise entre -10°C et 200°C, et à une pression comprise entre 0,01 MPa et 70 MPa.

Ledit photocatalyseur comprend au moins un semi-conducteur se présentant sous la forme d'un solide présentant une composition chimique exprimée sur une base anhydre, en termes de moles, par la formule générale suivante :

$$X_aY_bS_8\text{: cR}$$

où

X représente au moins un élément tétravalent choisi parmi Sn, Ge, Ti ou Zr,
Y représente au moins un métal divalent choisi parmi Zn, Cd ou Ni,
R représente au moins une espèce organique azotée,
S est le soufre,
« a » est la quantité molaire de X compris entre 0,1 et 5 ;
« b » est la quantité molaire de Y compris entre 0,2 et 8 ;
« c » est la quantité molaire de l'espèce organique azotée R compris entre 0 et 4.

Avantageusement, ledit photocatalyseur comprenant au moins un semi-conducteur se présente sous la forme d'un solide présentant une composition chimique exprimée sur une base anhydre, en termes de moles, définie par la formule générale suivante :

$$Sn_aZn_bS_8\text{ : cR}$$

où

R représente au moins une espèce organique azotée ;
S est le soufre ;
« a » est la quantité molaire du Sn compris entre 0,1 et 5 ;
« b » est la quantité molaire du Zn compris entre 0,2 et 8 ;
« c » est la quantité molaire de l'espèce organique azotée R compris entre 0 et 4.

Avantageusement, « c » est compris entre 0,2 et 4.

Avantageusement, ledit solide présente un diagramme de diffraction des rayons X incluant au moins les raies inscrites dans le tableau 1 ci-après :

| 2 thêta (°) | $d_{hkl}$ (Å) | $I_{rel}$ |
|---|---|---|
| 7,95 | 11,11 | F |
| 8,88 | 9,95 | m |
| 10,18 | 8,68 | f |
| 11,10 | 7,97 | FF |
| 11,72 | 7,54 | F |
| 15,37 | 5,76 | m |
| 16,71 | 5,30 | f |
| 17,36 | 5,11 | f |
| 21,49 | 4,13 | m |
| 22,30 | 3,98 | m |
| 23,31 | 3,81 | f |
| 30,05 | 2,97 | f |
| 33,34 | 2,69 | f |
| 35,96 | 2,50 | f |
| 40,80 | 2,21 | f |
| où FF = très fort ; F = fort ; m = moyen ; f = faible. | | |

Avantageusement, R est un composé organique comprenant au moins deux atomes d'azote.

Avantageusement, R est le 1,3-bis(4-piperidinyl)propane.

Avantageusement, ledit semi-conducteur présente un volume microporeux, déterminé par porosimétrie azote, compris entre 0,01 et 0,50 $cm^3/g$.

La largeur de la bande interdite dudit photocatalyseur est comprise entre 1,24 et 3 eV.

Dans un mode de réalisation selon l'invention, lorsque ledit procédé est effectué en phase gazeuse, le composé sacrificiel est un composé gazeux choisi parmi l'eau, l'ammoniaque, l'hydrogène, le méthane et un alcool.

Dans un mode de réalisation selon l'invention, dans lequel lorsque le procédé est effectué en phase liquide, le composé sacrificiel est un composé liquide ou solide soluble choisi parmi l'eau, l'ammoniaque, un alcool, un aldéhyde ou une amine.

Avantageusement, la source d'irradiation est une source d'irradiation naturelle.

De préférence, la source d'irradiation émet à au moins une gamme de longueur d'onde supérieure à 280 nm.

Plus préférentiellement, la source d'irradiation émet à au moins une gamme de longueur d'onde comprise entre 315 nm et 800 nm.

## Définitions et abréviations

Le terme « composé sacrificiel » correspond à un composé oxydable, sous forme gazeuse ou liquide.

On entend par « molécules carbonées en $C_1$ ou plus ($C_{1+}$)», des molécules issues de la réduction du $CO_2$

contenant un ou plus atomes de carbone, à l'exception du $CO_2$. De telles molécules sont par exemple le CO, le méthane, le méthanol, l'éthanol, le formaldéhyde, l'acide formique, le méthane ou encore d'autres molécules telles que les acides carboxyliques, les aldéhydes, les cétones, différents alcools ou des hydrocarbures contenant plus de 2 atomes de carbone.

Les groupes d'éléments chimiques correspondent à ceux de la classification CAS (CRC Handbook of Chemistry and Physics, éditeur CRC press, rédacteur en chef D.R. Lide, 81ème édition, 2000-2001). Par exemple, le groupe VIII selon la classification CAS correspond aux métaux des colonnes 8, 9 et 10 selon la nouvelle classification IUPAC.

Les propriétés texturales et structurales du support et du catalyseur décrits ci-après, sont déterminées par les méthodes de caractérisation connues de l'homme du métier.

Le volume microporeux et la distribution poreuse sont déterminés par porosimétrie à l'azote tel que décrit dans l'ouvrage « Adsorption by powders and porous solids. Principles, methodology and applications » écrit par F. Rouquérol, J. Rouquérol et K. Sing, Academic Press, 1999.

On entend par « surface spécifique », la surface spécifique BET ($S_{BET}$ en $m^2/g$) déterminée par adsorption d'azote conformément à la norme ASTM D 3663-78 établie à partir de la méthode BRUNAUER-EMMETT-TELLER décrite dans le périodique "The Journal of American Society", 1938, 60, 309.

On calcule la longueur d'onde maximale absorbable par un semi-conducteur à l'aide de l'équation suivante :

$$\lambda_{\max} = \frac{h \times c}{E_g}$$

Avec $\lambda_{max}$ la longueur l'onde maximale absorbable par un semi-conducteur (en m), h la constante de Planck ($4{,}13433559.10^{-15}$ eV.s), c la vitesse de la lumière dans le vide (299 792 458 $m.s^{-1}$) et Eg la largeur de bande interdite *("bandgap"* selon la terminologie anglo-saxonne) du semi-conducteur (en eV).

On entend par « milieu réactionnel », le mélange formé par la charge contenant le dioxyde de carbone, le composé sacrificiel et le photocatalyseur.

Dans la présente description, on entend, selon la convention IUPAC, par micropores les pores dont le diamètre est inférieur à 2 nm, c'est à dire 0,002 µm; par mésopores les pores dont le diamètre est supérieur à 2 nm, c'est à dire 0,002 µm et inférieur à 50 nm, c'est-à-dire 0,05 µm et par macropores les pores dont le diamètre est supérieur à 50nm, c'est-à-dire 0,05 µm.

## Description détaillée de l'invention

L'invention décrit un procédé de réduction photocatalytique du dioxyde de carbone effectué en phase liquide et/ou en phase gazeuse, ledit procédé comprenant les étapes suivantes :

a) on met en contact une charge contenant le dioxyde de carbone et au moins un composé sacrificiel avec un photocatalyseur comprenant au moins un semi-conducteur à base de sulfure métallique cristallisé microporeux ;
b) on irradie le photocatalyseur par au moins une source d'irradiation produisant au moins une longueur d'onde inférieure à la largeur de bande interdite dudit photocatalyseur, ladite étape b) étant réalisée à une température comprise entre -10°C et 200°C, et à une pression comprise entre 0,01 MPa et 70 MPa.

<u>Etape a) de mise en contact d'une charge, d'au moins un composé sacrificiel et d'un photocatalyseur à base de sulfure métallique cristallisé microporeux,</u>
Selon l'étape a) du procédé selon l'invention, on met en contact une charge contenant le dioxyde de carbone ($CO_2$) et au moins un composé sacrificiel avec un photocatalyseur à base de sulfure métallique cristallisé microporeux.

La mise en contact peut se faire par tout moyen connu de l'homme du métier. La mise en contact de la charge et du photocatalyseur peut se faire en lit fixe traversé, en lit fixe léchant ou en suspension (aussi appelé *"slurry"* selon la terminologie anglo-saxonne). Le photocatalyseur peut également être déposé directement sur des fibres optiques.

Lorsque la mise en contact est en lit fixe traversé, le photocatalyseur est préférentiellement déposé en couche sur un support poreux, par exemple de type fritté céramique ou métallique, et la charge contenant le dioxyde de carbone à convertir sous forme gazeuse et/ou liquide, est envoyée à travers le lit photocatalytique.

Lorsque la mise en en contact est en lit fixe léchant, le photocatalyseur est préférentiellement déposé sur un support non poreux de type céramique ou métallique, et la charge contenant le dioxyde de carbone à convertir sous forme gazeuse et/ou liquide, est envoyée sur le lit photocatalytique.

Lorsque la mise en contact est en suspension, le photocatalyseur est préférentiellement sous forme de particules en suspension dans une charge liquide ou liquide-gaz contenant le dioxyde de carbone. **En** suspension, la mise en œuvre peut se faire dans un réacteur fermé ou en continu.

*- La charge et les composés sacrificiels*

Le procédé est effectué en phase gazeuse, liquide ou biphasique, gazeuse et liquide, signifiant respectivement que la charge traitée selon le procédé se présente sous forme gazeuse, liquide ou biphasique, gazeuse et liquide. De préférence, le procédé est effectué en phase gazeuse.

Lorsque le procédé est effectué en phase gazeuse, avec une charge se présentant sous forme gazeuse, le $CO_2$ présent dans la charge est aussi sous forme gazeuse, et le(s) composé(s) sacrificiel(s) utilisés pour l'étape a) sont également sous forme gazeuse.

Les composés sacrificiels gazeux sont des composés oxydables tels que l'eau ($H_2O$), l'ammoniaque ($NH_3$), le dihydrogène ($H_2$), le méthane ($CH_4$) ou encore les alcools, ou leur mélange. De manière préférée, les composés sacrificiels gazeux sont l'eau ou le dihydrogène. Un fluide diluant tel que $N_2$ ou Ar, peut être présent dans le milieu réactionnel lorsque le procédé est effectué en phase gazeuse. La présence d'un fluide diluant n'est pas requise pour la réalisation de l'invention, cependant il peut être utile d'en adjoindre à la charge pour assurer la dispersion de la charge et/ou du photocatalyseur dans le milieu réactionnel, le contrôle de l'adsorption des réactifs/produits à la surface du photocatalyseur, le contrôle de l'absorption des photons par le photocatalyseur, la dilution des produits pour limiter leur recombinaison et autres réactions parasites du même ordre. La présence d'un fluide diluant permet aussi le contrôle de la température du milieu réactionnel pouvant ainsi compenser l'éventuelle exo/endo-thermicité de la réaction photocatalysée. La nature du fluide diluant est choisie de telle façon que son influence soit neutre sur le milieu réactionnel ou que son éventuelle réaction ne nuise pas à la réalisation de la réduction souhaitée du dioxyde de carbone.

Lorsque le procédé est effectué en phase liquide, avec une charge se présentant sous forme liquide, celle-ci peut être sous forme ionique, organique ou aqueuse. La charge sous forme liquide est préférentiellement aqueuse.

Lorsque la charge liquide est une solution aqueuse, le $CO_2$ est alors solubilisé sous forme de $CO_2$ aqueux, d'hydrogénocarbonate ou de carbonate. Les composés sacrificiels utilisés dans ce cas, sont des composés oxydables liquides ou solides solubles dans la charge liquide, tels que l'eau ($H_2O$), l'ammoniaque ($NH_3$), les alcools, les aldéhydes, les amines. De manière préférée, le composé sacrificiel est l'eau. Le pH est généralement compris entre 2 et 12, de préférence entre 3 et 10.

Eventuellement, et afin de moduler le pH de la charge liquide aqueuse, un agent basique ou acide peut être ajouté à la charge. L'agent basique peut être choisi parmi les hydroxydes d'alcalins ou d'alcalinoterreux, les bases organiques, par exemple les amines ou l'ammoniaque. L'agent acide peut être choisi parmi les acides inorganiques, par exemple l'acide nitrique, sulfurique, phosphorique, chlorhydrique, bromhydrique ou les acides organiques, tels que des acides carboxyliques ou sulfoniques.

Eventuellement, lorsque la charge liquide est aqueuse, celle-ci peut contenir en toute quantité tout ion solvaté, tels que par exemple $K^+$, $Li^+$, $Na^+$, $Ca^{2+}$, $Mg^{2+}$, $SO_4^{2-}$, $Cl^-$, $F^-$, $NO_3^{2-}$

*- Le photocatalyseur*

Le photocatalyseur comprend, de préférence est constitué de, un ou de plusieurs semi-conducteurs à base de sulfure métallique cristallisé microporeux.

Ledit semi-conducteur se présente sous la forme d'un solide comprenant une composition chimique exprimée sur une base anhydre, en termes de moles, par la formule générale suivante :

$$X_aY_bS_8 : cR$$

où

X représente au moins un élément tétravalent choisi parmi Sn, Ge, Ti ou Zr,
Y représente au moins un métal divalent choisi parmi Zn, Cd ou Ni,
R représente au moins une espèce organique azotée,
S est le soufre,
« a » est la quantité molaire de X compris entre 0,1 et 5 ;
« b » est la quantité molaire de Y compris entre 0,2 et 8 ;
« c » est la quantité molaire de l'espèce organique azotée R compris entre 0 et 4.

Plus préférentiellement, ledit photocatalyseur comprend un solide, appelé IZM-5, ledit solide IZM-5 présentant une composition chimique exprimée sur une base anhydre, en termes de moles, définie par la formule générale suivante :

$$Sn_aZn_bS_8 : cR$$

où

R représente au moins une espèce organique azotée ;
S est le soufre ;
« a » est la quantité molaire du Sn compris entre 0,1 et 5, de préférence entre 1 et 4 ;
« b » est la quantité molaire du Zn compris entre 0,2 et 8, de préférence entre 0,2 et 2 ;
« c » est la quantité molaire de l'espèce organique azotée R compris entre 0 et 4, de préférence entre 0,5 et 3.

De préférence, R comporte deux atomes d'azote, et très préférentiellement R est le 1,3-bis(4-piperidinyl) propane dont la formule développée est donnée ci-dessous.

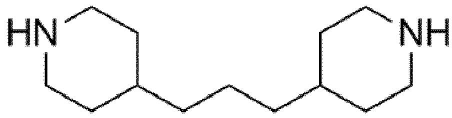

Le semi-conducteur constitutif dudit photocatalyseur est cristallisé et présente un signal en diffraction des rayons X bien spécifique. Plus préférentiellement, le photocatalyseur comprend le solide IZM-5 présentant un diagramme de diffraction de rayons X incluant au moins les raies inscrites dans le tableau 1 ci-avant.

L'intensité relative $I_{rel}$ est donnée en rapport à une échelle d'intensité relative où il est attribué une valeur de 100 à la raie la plus intense du diagramme de diffraction des rayons X : $5 \leq f < 10$ ; $10 \leq m < 15$ ; $15 \leq F < 50$ ; $FF \geq 50$.

Ce diagramme de diffraction est obtenu par analyse radiocristallographique au moyen d'un diffractomètre en utilisant la méthode classique des poudres avec le rayonnement $K\alpha_1$ du cuivre ($\lambda$ = 1,5406Å). A partir de la position des pics de diffraction représentée par l'angle $2\theta$, on calcule, par la relation de Bragg, les équidistances interréticulaires $d_{hkl}$ caractéristiques de l'échantillon. L'erreur de mesure $\Delta(d_{hkl})$ sur $d_{hkl}$ est calculée grâce à la relation de Bragg en fonction de l'erreur absolue $\Delta(2\theta)$ affectée à la mesure de $2\theta$. Une erreur absolue $\Delta(2\theta)$ égale à $\pm$ 0,02° est communément admise. L'intensité relative $I_{rel}$ affectée à chaque valeur de $d_{hkl}$ est mesurée d'après la hauteur du pic de diffraction correspondant. Dans la colonne des $d_{hkl}$, on a indiqué les valeurs moyennes des distances inter-réticulaires en Angströms (Å). Chacune de ces valeurs doit être affectée de l'erreur de mesure $\Delta(d_{hkl})$ comprise entre $\pm$ 0,6Å et $\pm$ 0,01Å.

Le semi-conducteur constitutif dudit photocatalyseur présente de préférence un volume microporeux, déterminé par porosimétrie azote, compris entre 0,01 et 0,50 $cm^3/g$, de préférence entre 0,05 et 0,30 $cm^3/g$.

La largeur de bande interdite dudit photocatalyseur est comprise entre 1,24 et 3 eV.

Le photocatalyseur peut éventuellement être dopé avec un ou plusieurs ions choisis parmi des ions métalliques, tels que par exemple des ions de V, Ni, Cr, Mo, Fe, Sn, Mn, Co, Re, Nb, Sb, La, Ce, Ta, Ti, des ions non-métalliques, tels que par exemple des ions C, N, S, F, P, ou par un mélange d'ions métalliques et non-métalliques.

Le photocatalyseur peut éventuellement contenir des particules comportant un ou plusieurs élément(s) à l'état métallique, choisis parmi un élément des groupes IVB, VB, VIB, VIIB, VIIIB, IB, IIB, IIIA, IVA et VA de la classification périodique des éléments. Lesdites particules comportant un ou plusieurs élément(s) à l'état métallique sont en contact direct avec ledit semi-conducteur. Lesdites particules peuvent être composées d'un seul élément à l'état métallique ou de plusieurs éléments à l'état métallique pouvant former un alliage. On entend par « élément à l'état métallique » (à ne pas confondre avec « élément métallique ») un élément appartenant à la famille des métaux, ledit élément étant au degré d'oxydation zéro (et donc sous forme de métal). De préférence, le ou les éléments à l'état métallique sont choisis parmi un élément métallique des groupes VIIB, VIIIB, IB et IIB de la classification périodique des éléments, et de manière particulièrement préférée, parmi le platine, le palladium, l'or, le nickel, le cobalt, le ruthénium, l'argent, le cuivre, le rhénium ou le rhodium. Lesdites particules comportant un ou plusieurs élément(s) à l'état métallique se présentent préférentiellement sous la forme de particules de tailles comprises entre 0,5 nm et 1000 nm, de manière préférée entre 0,5 nm et 100 nm et encore plus préférentiellement entre 1 et 20 nm.

Le photocatalyseur utilisé dans le procédé selon l'invention peut se présenter sous différentes formes ou mises en formes (poudre nanométrique, nanoobjets comportant ou non des cavités, films, monolithe, billes de taille micrométrique ou millimétrique,...). Le photocatalyseur se présente avantageusement sous forme de poudre nanométrique.

*- Le procédé de préparation du photocatalyseur*

Le semi-conducteur constitutif dudit photocatalyseur peut être obtenu par le procédé de préparation tel que décrit ci-après.

i) on mélange au moins une source d'élément tétravalent noté X, au moins une source de métal divalent noté Y, au moins une source de soufre noté S, au moins une espèce organique noté R, éventuellement au moins un solvant noté

SOLV, comprenant au moins un composé aqueux (noté A) et/ou au moins un composé organique (noté O), pour obtenir un gel précurseur, ledit mélange présentant préférentiellement la composition molaire suivante :

X/Y : au moins 0,1, de préférence de 1 à 200,
S/(X + Y) : 0,05 à 50, de préférence de 0,1 à 20,
R/(X + Y) : 0,05 à 50, de préférence de 0,1 à 20,
SOLV/(X + Y) : 0 à 200, de préférence de 1 à 100,
A/O : 0,005 à 100, de préférence de 0,1 à 20,

ii) on réalise un traitement thermique dudit gel précurseur obtenue à l'issue de l'étape i) à une température comprise entre 120°C et 250°C, pendant une durée comprise entre 2 jours et 21 jours.

Le procédé de préparation consiste à préparer un mélange réactionnel appelé gel et renfermant au moins une source d'élément tétravalent noté X, une source de métal divalent noté Y, une source de soufre noté S, au moins une espèce organique R, et éventuellement un solvant. Les quantités desdits réactifs sont ajustées de manière à conférer à ce gel une composition permettant sa cristallisation en solide cristallisé sous sa forme brute de synthèse de formule générale $X_aY_bS_8$: cR où a, b et c répondent aux critères définis plus haut.

*Etape i)*

L'étape i) de mélange est mise en œuvre jusqu'à obtention d'un mélange homogène, de préférence pendant une durée supérieure ou égal à 15 minutes, de préférence sous agitation par tout système connu de l'homme du métier à faible ou fort taux de cisaillement. A l'issue de l'étape i) on obtient un gel précurseur homogène.

Il peut être avantageux d'additionner des germes au mélange réactionnel au cours de ladite étape i) du procédé selon l'invention afin de réduire le temps nécessaire à la formation des cristaux et/ou la durée totale de cristallisation. Lesdits germes favorisent également la formation du solide cristallisé au détriment d'impuretés. De tels germes comprennent des solides cristallisés, notamment des cristaux de solide. Les germes cristallins sont généralement ajoutés dans une proportion comprise entre 0,01% et 10 % en poids par rapport au poids total des précurseurs d'étain et de zinc utilisés dans le mélange réactionnel.

Il peut être avantageux de mettre en oeuvre un mûrissement du mélange réactionnel avant le traitement thermique au cours de ladite étape i) du procédé selon l'invention afin de contrôler la taille des cristaux du solide cristallisé. Ledit mûrissement favorise également la formation de ledit solide cristallisé au détriment d'impuretés. Le murissement du mélange réactionnel au cours de ladite étape i) du procédé selon l'invention peut être réalisé à température ambiante ou à une température comprise entre 20°C et 100°C avec ou sans agitation, pendant une durée avantageusement comprise entre 30 min et 48 heures.

Lorsque le photocatalyseur comprend le solide IZM-5, ledit solide IZM-5 est obtenu en faisant réagir un mélange comportant au moins une source d'étain noté Sn, au moins une source de zinc noté Zn, au moins une source de soufre noté S, au moins une espèce organique azotée noté R, éventuellement au moins un solvant noté SOLV, comprenant au moins un composé aqueux (noté A) et/ou au moins un composé organique (noté O), le mélange présentant préférentiellement la composition molaire suivante :

Sn/Zn : au moins 0,1, de préférence au moins 1, de manière plus préférée de 2 à 200,
S/(Sn + Zn) : 0,1 à 20, de préférence de 1 à 10,
R/(Sn + Zn) : 0,1 à 10, de préférence de 1 à 5,
SOLV/(Sn + Zn) : 0 à 200, de préférence de 10 à 100, de manière plus préférée de 20 à 100,
A/O : 0,01 à 10, de préférence de 0,1 à 8, de préférence de 0,2 à 5.

R est une espèce organique azotée ayant au moins un atome d'azote, de préférence R comporte deux atomes d'azote, jouant le rôle de structurant organique. Préférentiellement, R est le composé azoté 1,3-bis(4-piperidinyl)propane. Ladite espèce organique azotée utilisée comme agent structurant du solide cristallisé IZM-5 est synthétisée par toute méthode connue de l'Homme du métier.

La source d'étain, employée pour la mise en oeuvre du procédé de préparation du solide cristallisé IZM-5, peut être tout composé comprenant l'élément étain et pouvant libérer cet élément dans le mélange sous forme réactive. La source d'étain est de préférence l'acétate d'étain $Sn(CH_3CO_2)_4$, le tert-butoxyde d'étain $Sn(OC(CH_3)_3)_4$, le tetrachlorure d'étain $SnCl_4$, le bis(acetylacetonate) dichlorure d'étain $(CH_3COCH=C\text{-}(O\text{-})CH_3)_2SnCl_2$, l'oxyde d'étain $SnO_2$, l'étain sous forme métallique Sn.

La source de zinc, employée pour la mise en oeuvre du procédé de préparation du solide cristallisé IZM-5, peut être tout composé comprenant l'élément zinc et pouvant libérer cet élément dans le mélange sous forme réactive. La

source de zinc est de préférence le chlorure de zinc $ZnCl_2$, l'acétate de zinc $Zn(CH_3CO_2)_2$, le sulfate de zinc $ZnSO_4$, le nitrate de zinc $Zn(NO_3)_2$, l'oxyde de zinc ZnO, le zinc sous forme métallique Zn.

La source de soufre, employée pour la mise en oeuvre du procédé de préparation du solide cristallisé IZM-5, peut être tout composé comprenant l'élément soufre et pouvant libérer cet élément dans le mélange sous forme réactive. La source de soufre est de préférence solide ou liquide dans les conditions normale de température et de pression. La source de soufre est de préférence du soufre élémentaire S ou $S_8$, le sulfure de sodium $Na_2S$, le sulfure de potassium $K_2S$, le sulfure de lithium $Li_2S$, le sulfure d'ammonium $S(NH_4)_2$, le diméthyle disulfure $CH_3SSCH_3$.

Le solvant, employé pour la mise en oeuvre du procédé de préparation du solide cristallisé IZM-5, peut être un composé aqueux et/ou organique. Selon une variante, le solvant est constitué d'un composé aqueux et d'un composé organique. Le composé aqueux est de préférence choisi parmi l'eau $H_2O$, et le composé organique est choisi de préférence par les composés liquides aux conditions normales de température et de pression de type alcool (de préférence éthanol, iso-propanol), diol (de préférence éthylène glycol, propylène glycol), triol (de préférence glycérol ou propane-1,2,3-triol), organosulfurés (de préférence diméthylsulfoxyde ou DMSO), organonitrés (de préférence le diméthylformamide ou DMF).

Selon une autre variante, il n'est pas utilisé de solvant additionnel dans le procédé de préparation du solide cristallisé IZM-5, et c'est l'espèce organique azotée notée R lorsqu'elle est sous forme liquide aux conditions normales de température et de pression qui permet la solubilisation des précurseurs métalliques et précurseurs soufrés.

*Etape ii)*

Conformément à l'étape ii) du procédé de préparation, gel obtenu à l'issue de l'étape i) est soumis à un traitement thermique, préférentiellement réalisé à une température comprise entre 120°C et 250°C pendant une durée comprise entre 2 jours et 21 jours jusqu'à ce que le solide cristallisé se forme.

Le gel est avantageusement mis sous une pression de réaction autogène, éventuellement en ajoutant du gaz, par exemple de l'azote, à une température comprise entre 120°C et 250°C, de préférence entre 140°C et 210°C jusqu'à la formation des cristaux de solide sous sa forme brute de synthèse.

La durée nécessaire pour obtenir la cristallisation varie généralement entre 1 jour et plusieurs mois en fonction de la composition des réactifs dans le gel, de l'agitation et de la température de réaction. De préférence la durée de cristallisation varie entre 2 jours et 21 jours et de préférence entre 5 jours et 15 jours.

La mise en réaction s'effectue généralement sous agitation ou en absence d'agitation, de préférence sous d'agitation. Comme système d'agitation on peut utiliser tout système connu par l'homme de métier, par exemple, des pales inclinées avec des contre pales, des turbines d'agitation, des vis d'Archimède.

A l'issue de l'étape de traitement thermique conduisant à la cristallisation du solide, la phase solide est de préférence filtrée, lavée puis séchée. De manière préférée, l'étape de lavage sera effectuée avec de l'éthanol ou avec le solvant utilisé pour la synthèse.

Avantageusement, à l'issue de l'étape ii) de traitement thermique, éventuellement à l'issue des étapes de filtrage, lavage et séchage telles que décrites ci-avant, on réalise une étape d'extraction de l'espèce organique R afin de libérer la microporosité par toute méthode connue de l'Homme du métier. De préférence, cette étape peut être réalisée à l'aide de traitement thermique de 100°C à 1000°C sous air, sous oxygène, sous hydrogène, sous $H_2S$, ou encore sous gaz inerte tel que $N_2$, seuls ou en mélange. Cette extraction peut également se faire par échange ionique avec des espèces telles que $NH_4^+$, des alcalins, alcalino-terreux ou tout cation métallique.

Etape b) d'irradiation du photocatalyseur

Selon l'étape b) du procédé selon l'invention, on irradie le photocatalyseur par au moins une source d'irradiation produisant au moins une longueur d'onde absorbable par le photocatalyseur (soit inférieure à la largeur de bande interdite du semi-conducteur constitutif dudit photocatalyseur selon la variante où le photocatalyseur est composé d'au moins un semi-conducteur) de manière à réduire le dioxyde de carbone et oxyder le composé sacrificiel en présence dudit photocatalyseur activé par ladite source d'irradiation, de manière à produire un effluent contenant au moins en partie des molécules carbonées en C1 ou plus, différentes du $CO_2$.

Un photocatalyseur comprenant un ou de plusieurs semi-conducteurs à base de sulfure métallique cristallisé microporeux, peut être activé par l'absorption d'au moins un photon.

Les photons absorbables sont ceux dont l'énergie est supérieure à la largeur de bande interdite, au "bandgap". Autrement dit, les photocatalyseurs sont activables par au moins un photon d'une longueur d'onde correspondant à l'énergie associée aux largeurs de bandes interdites des semi-conducteurs constituant le photocatalyseur ou d'une longueur d'onde inférieure.

Toute source d'irradiation émettant au moins une longueur d'onde adaptée à l'activation dudit photocatalyseur c'est-à-dire absorbable par le photocatalyseur peut être utilisée selon l'invention. La source d'irradiation peut être aussi

bien naturelle par irradiation solaire qu'artificielle de type laser, Hg, lampe à incandescence, tube fluorescent, plasma ou diode électroluminescente (DEL, ou **LED** en anglais pour Light-Emitting Diode). De manière préférée, la source d'irradiation est naturelle, de préférence par irradiation solaire.

La source d'irradiation produit un rayonnement dont au moins une partie des longueurs d'onde est inférieure à la longueur d'onde maximale absorbable ($\lambda_{max}$) par les semi-conducteurs constitutifs du photocatalyseur selon l'invention. Lorsque la source d'irradiation est l'irradiation solaire, elle émet généralement dans le spectre ultra-violet, visible et infrarouge, c'est-à-dire elle émet une gamme de longueur d'onde de 280 nm à 2500 nm environ (selon la norme ASTM G173-03). De préférence, la source émet à au moins une gamme de longueur d'onde supérieure à 280 nm, de manière très préférée comprise entre 315 nm et 800 nm, ce qui inclut le spectre UV et/ou le spectre visible.

La source d'irradiation fournit un flux de photons qui irradie le milieu réactionnel contenant le photocatalyseur. L'interface entre le milieu réactionnel et la source lumineuse varie en fonction des applications et de la nature de la source lumineuse.

Lorsque la source d'irradiation est naturelle, par exemple une irradiation solaire, la source d'irradiation est localisée à l'extérieur du réacteur et l'interface entre les deux peut être une fenêtre optique en pyrex, en quartz, en verre organique ou toute autre interface permettant aux photons absorbables par le photocatalyseur selon l'invention, de diffuser du milieu extérieur au sein du réacteur.

La réalisation de la réduction photocatalytique du dioxyde de carbone est conditionnée par la fourniture de photons adaptés au système photocatalytique pour la réaction envisagée et de ce fait n'est pas limitée à des gammes de pression ou de température spécifiques en dehors de celles permettant d'assurer la stabilité du ou des produit(s). La gamme de température employée pour la réduction photocatalytique de la charge contenant le dioxyde de carbone est généralement de -10°C à + 200°C, de manière préférée de 0 à 150°C, et de manière très préférée de 0 et 50 °C. La gamme de pression employée pour la réduction photocatalytique de la charge contenant le dioxyde de carbone est généralement de 0,01 MPa à 70 MPa (0,1 à 700 bar), de manière préférée de 0,1 MPa à 5 MPa (1 à 50 bar).

Un fluide diluant tel que décrit dans l'étape a), peut être présent dans le milieu réactionnel lorsque le procédé est effectué en phase gazeuse, durant l'irradiation.

L'effluent obtenu après la réaction de réduction photocatalytique du dioxyde de carbone contient d'une part au moins une molécule en $C_1$ ou plus, différente du dioxyde de carbone issue de la réaction et d'autre part de la charge non réagie, ainsi que l'éventuel fluide diluant, mais aussi des produits de réactions parallèles tels que le dihydrogène résultant de la réduction photocatalytique d'$H_2O$ lorsque ce composé est utilisé en tant que composé sacrificiel.

Les exemples suivants illustrent l'invention sans en limiter la portée.

## Exemples

Exemple 1 : Photocatalyseur A - $TiO_2$

Le photocatalyseur A est un semi-conducteur à base de $TiO_2$ commercial (Aeroxide® P25, Aldrich™, pureté > 99,5%). La granulométrie du photocatalyseur mesurée par microscopie électronique à transmission (MET) est de 21 nm et la surface spécifique mesurée par méthode BET est égale à 52 $m^2$/g.

Le photocatalyseur A possède une largeur de bande interdite de 3,1 eV mesurée par spectrométrie UV-Visible en réflexion diffuse et ne présente pas de microporosité.

Exemple 2 : Préparation d'un solide IZM-5

0,228 g de dioxyde d'étain ($SnO_2$, pureté 99% en poids, Sigma-Aldrich) ont été mélangés avec 0,146 g de nitrate de zinc ($Zn(NO_3)_2 \cdot 6H_2O$, pureté 99% en poids, Alfa Aesar). Par la suite, 0,277 g de soufre (S, pureté 99,98% en poids, Aldrich) et 2,001 g de 1,3-bis(4-piperydil)propane (composé R, pureté 97% en poids, Aldrich) sont ajoutés au mélange précédent. Au final, 11,2 mL d'éthylène glycol (pureté 99,8% en poids, VWR) et 3,7 mL d'eau déionisée sont incorporés et le gel de synthèse est maintenu sous agitation (250 tr/min) pendant 30 minutes. Le gel précurseur est ensuite transféré, après homogénéisation dans un autoclave. L'autoclave est fermé puis chauffé pendant 12 jours à 190°C dans des conditions statiques. Le produit cristallisé obtenu est filtré, lavé à l'éthanol puis séché une nuit à 100°C. Le produit solide brut de synthèse a été analysé par diffraction des rayons X et identifié comme étant constitué de solide IZM-5. Le produit présente un rapport molaire Sn/Zn de 5,7 tel que déterminé par ICP-MS. L'analyse élémentaire donne la composition molaire suivante : $Sn_{3.4}Zn_{0.6}S_8$ :1,1R.

Exemple 3 : Photocatalyseur B

On soumet le solide obtenu à l'exemple 2 a un traitement thermique pour extraire tout ou partie du composé R afin de libérer la microporosité. Un mélange à 50% volumique $N_2$ et 50% volumique air à un débit de 2 NL/h/g est introduit dans

un réacteur de type lit traversé contenant le solide IZM-5 obtenu à l'exemple 2. La température est augmentée à 120°C à une vitesse de 1°C/min, puis laissé pendant 1h. Dans un second temps, la température est augmentée à 300°C à une vitesse de 1°C/min, puis laissé pendant 2h. On laisse enfin la température redescendre à l'ambiante par l'inertie du réacteur.

Le solide récupéré est nommé photocatalyseur B. Le photocatalyseur B possède une largeur de bande interdite de 2,6 eV mesurée par spectrométrie UV-Visible en réflexion diffuse et présente un volume microporeux de 0,1 mL/g.

Exemple 4 : Mise en oeuvre des photocatalyseurs A et B en réduction photocatalytique du $CO_2$ en phase gazeuse

Les solides A et B sont soumis à un test de réduction photocatalytique du $CO_2$ en phase gazeuse dans un réacteur continu à lit traversé en acier muni d'une fenêtre optique en quartz d'une surface de $5,3.10^{-4}$ $m^2$ et d'un fritté en face de la fenêtre optique sur lequel est déposé le solide photocatalytique.

Environ 100 mg de photocatalyseur sont déposés sur le fritté. Les tests sont réalisés à température ambiante sous pression atmosphérique. Un débit de $CO_2$ de 18 ml/h traverse un saturateur d'eau avant d'être distribué dans le réacteur. On suit la production de $CH_4$ et de CO issu de la réduction du dioxyde de carbone, par une analyse de l'effluent toutes les 6 minutes par micro chromatographie en phase gazeuse. La source d'irradiation UV-Visible est fournie par une lampe Xe-Hg (Asahi™, MAX302™). La puissance d'irradiation est toujours maintenue à 130 $W/m^2$ mesurée pour une gamme de longueur d'onde comprise entre 315 nm et 400 nm. La durée du test est de 20 heures.

La comparaison des activités photocatalytiques moyennes sont exprimées en $\mu$mol de méthane ou de monoxyde de carbone produits par heure et par surface d'irradiation. Les résultats sont reportés dans le tableau 2 ci-après. Les valeurs d'activité montrent que la mise en oeuvre des solides selon l'invention présente les meilleures performances photocatalytiques.

| Photocatalyseur | Production de $CH_4$ ($\mu$mol/h/$m^2$) | Production de CO ($\mu$mol/h/$m^2$) |
|---|---|---|
| A (comparatif) | 1,0 | 1,3 |
| B (selon l'invention) | 40 | 110 |

## Revendications

1. Procédé de réduction photocatalytique du dioxyde de carbone effectué en phase liquide et/ou en phase gazeuse, ledit procédé comprenant les étapes suivantes :

   a) on met en contact une charge contenant le dioxyde de carbone et au moins un composé sacrificiel avec un photocatalyseur comprenant au moins un semi-conducteur à base de sulfure métallique cristallisé microporeux sous la forme d'un solide comprenant une composition chimique exprimée sur une base anhydre, en termes de moles, par la formule générale suivante :

   $$X_aY_bS_8 : cR$$

   où

   X représente au moins un élément tétravalent choisi parmi Sn, Ge, Ti ou Zr,
   Y représente au moins un métal divalent choisi parmi Zn, Cd ou Ni,
   R représente au moins une espèce organique azotée,
   S est le soufre,
   « a » est la quantité molaire de X compris entre 0,1 et 5 ;
   « b » est la quantité molaire de Y compris entre 0,2 et 8 ;
   « c » est la quantité molaire de l'espèce organique azotée R compris entre 0 et 4 ;

   b) on irradie le photocatalyseur par au moins une source d'irradiation produisant au moins une longueur d'onde inférieure à la largeur de bande interdite dudit photocatalyseur, comprise entre 1,24 et 3 eV, ladite étape b) étant réalisée à une température comprise entre -10°C et 200°C, et à une pression comprise entre 0,01 MPa et 70 MPa.

2. Procédé selon la revendication 1, dans lequel ledit photocatalyseur comprenant au moins un semi-conducteur se présente sous la forme d'un solide comprenant une composition chimique exprimée sur une base anhydre, en termes

de moles, définie par la formule générale suivante :

$$Sn_aZn_bS_8 : cR$$

où

R représente au moins une espèce organique azotée ;
S est le soufre ;
« a » est la quantité molaire du Sn compris entre 0,1 et 5 ;
« b » est la quantité molaire du Zn compris entre 0,2 et 8 ;
« c » est la quantité molaire de l'espèce organique azotée R compris entre 0 et 4.

**3.** Procédé selon l'une des revendications 1 ou 2, dans lequel « c » est compris entre 0,2 et 4.

**4.** Procédé selon la revendication 3, dans lequel ledit solide présentant un diagramme de diffraction des rayons X incluant au moins les raies inscrites dans le tableau ci-après :

| 2 thêta (°) | $d_{hkl}$ (Å) | $I_{rel}$ |
|---|---|---|
| 7,95 | 11,11 | F |
| 8,88 | 9,95 | m |
| 10,18 | 8,68 | f |
| 11,10 | 7,97 | FF |
| 11,72 | 7,54 | F |
| 15,37 | 5,76 | m |
| 16,71 | 5,30 | f |
| 17,36 | 5,11 | f |
| 21,49 | 4,13 | m |
| 22,30 | 3,98 | m |
| 23,31 | 3,81 | f |
| 30,05 | 2,97 | f |
| 33,34 | 2,69 | f |
| 35,96 | 2,50 | f |
| 40,80 | 2,21 | f |
| où FF = très fort ; F = fort ; m = moyen ; f = faible. | | |

**5.** Procédé selon l'une quelconque des revendications 1 à 4, dans lequel R est un composé organique comprenant au moins deux atomes d'azote.

**6.** Procédé selon la revendication 5, dans lequel R est le 1,3-bis(4-piperidinyl)propane.

**7.** Procédé selon l'une quelconque des revendications 1 à 6, dans lequel ledit semi-conducteur présente un volume microporeux, déterminé par porosimétrie à l'azote , compris entre 0,01 et 0,50 $cm^3/g$.

**8.** Procédé selon l'une quelconque des revendications 1 à 7, dans lequel lorsque ledit procédé est effectué en phase gazeuse, le composé sacrificiel est un composé gazeux choisi parmi l'eau, l'ammoniaque, l'hydrogène, le méthane et un alcool.

**9.** Procédé selon l'une quelconque des revendications 1 à 7, dans lequel lorsque le procédé est effectué en phase liquide, le composé sacrificiel est un composé liquide ou solide soluble choisi parmi l'eau, l'ammoniaque, un alcool, un aldéhyde ou une amine.

**10.** Procédé selon l'une quelconque des revendications 1 à 9, dans lequel la source d'irradiation est une source d'irradiation naturelle.

**11.** Procédé selon l'une quelconque des revendications 1 à 10, dans lequel la source d'irradiation émet à au moins une gamme de longueur d'onde supérieure à 280 nm.

**12.** Procédé selon la revendication 11, dans lequel la source d'irradiation émet à au moins une gamme de longueur d'onde comprise entre 315 nm et 800 nm.

**Patentansprüche**

**1.** Verfahren zur photokatalytischen Reduktion von Kohlendioxid, dass in der Flüssigphase und/oder in der Gasphase durchgeführt wird, wobei das Verfahren die folgenden Schritte umfasst:

a) Inkontaktbringen eines Einsatzstoffs, der Kohlendioxid und mindestens eine Opferverbindung umfasst, mit einem Photokatalysator, umfassend mindestens einen Halbleiter auf der Basis von mikroporösem kristallinem Metallsulfid in Form eines Feststoffs mit einer chemischen Zusammensetzung, die auf wasserfreier Basis in Mol durch die folgende allgemeine Formel ausgedrückt wird:

$$X_aY_bS_8 : cR$$

wobei

X für mindestens ein vierwertiges Element steht, das aus Sn, Ge, Ti oder Zr ausgewählt ist,
Y für mindestens ein zweiwertiges Metall steht, das aus Zn, Cd oder Ni ausgewählt ist,
R für mindestens eine stickstoffhaltige organische Spezies steht;
S Schwefel ist,
"a" die molare Menge von X zwischen 0,1 und 5 ist;
"b" die molare Menge von Y zwischen 0,2 und 8 ist;
"c" die molare Menge der stickstoffhaltigen organischen Spezies R zwischen 0 und 4 ist;

b) Bestrahlen des Fotokatalysators mit mindestens einer Bestrahlungsquelle, die mindestens eine Wellenlänge erzeugt, die kleiner ist als die Bandlücke des Photokatalysators, die zwischen 1,24 und 3 eV liegt, wobei der Schritt b) bei einer Temperatur zwischen -10 °C und 200 °C und einem Druck zwischen 0,01 MPa und 70 MPa durchgeführt wird.

**2.** Verfahren nach Anspruch 1, wobei der Photokatalysator, der mindestens einen Halbleiter umfasst, in Form eines Feststoffs mit einer chemischen Zusammensetzung, die auf wasserfreier Basis in Mol durch die folgende allgemeine Formel definiert ist, vorliegt:

$$Sn_aZn_bS_8: cR$$

wobei

R für mindestens eine stickstoffhaltige organische Spezies steht;
S Schwefel ist;
"a" die molare Menge von Sn zwischen 0,1 und 5 ist;
"b" die molare Menge von Zn zwischen 0,2 und 8 ist;
"c" die molare Menge der stickstoffhaltigen organischen Spezies R zwischen 0 und 4 ist.

**3.** Verfahren nach Anspruch 1 oder 2, wobei "c" zwischen 0,2 und 4 liegt.

**4.** Verfahren nach Anspruch 3, wobei der Feststoff ein Röntgenbeugungsdiagramm aufweist, das mindestens die in der folgenden Tabelle aufgeführten Linien umfasst:

| 2 Theta (°) | $d_{hkl}$ (Å) | $I_{rel}$ |
|---|---|---|
| 7, 95 | 11,11 | S |
| 8, 88 | 9, 95 | m |
| 10, 18 | 8, 68 | s |
| 11, 10 | 7, 97 | SS |
| 11,72 | 7, 54 | S |
| 15, 37 | 5,76 | m |
| 16, 71 | 5,30 | s |
| 17,36 | 5,11 | s |
| 21, 49 | 4,13 | m |
| 22,30 | 3, 98 | m |
| 23,31 | 3,81 | s |
| 30,05 | 2,97 | s |
| 33,34 | 2,69 | s |
| 35,96 | 2,50 | s |
| 40,80 | 2,21 | s |
| wobei SS = sehr stark; S = stark; m = mittel; s = schwach. | | |

**5.** Verfahren nach einem der Ansprüche 1 bis 4, wobei R eine organische Verbindung mit mindestens zwei Stickstoffatomen ist.

**6.** Verfahren nach Anspruch 5, wobei R 1,3-Bis(4-piperidinyl)propan ist.

**7.** Verfahren nach einem der Ansprüche 1 bis 6, wobei der Halbleiter ein durch Stickstoffporosimetrie bestimmtes Mikroporenvolumen zwischen 0,01 und 0,50 $cm^3/g$ aufweist.

**8.** Verfahren nach einem der Ansprüche 1 bis 7, wobei es sich dann, wenn das Verfahren in der Gasphase durchgeführt wird, bei der Opferverbindung um eine gasförmige Verbindung handelt, die aus Wasser, Ammoniak, Wasserstoff, Methan und einem Alkohol ausgewählt wird.

**9.** Verfahren nach einem der Ansprüche 1 bis 7, wobei es sich dann, wenn das Verfahren in der Flüssigphase durchgeführt wird, bei der Opferverbindung um eine lösliche flüssige oder feste Verbindung handelt, die aus Wasser, Ammoniak, einem Alkohol, einem Aldehyd oder einem Amin ausgewählt wird.

**10.** Verfahren nach einem der Ansprüche 1 bis 9, wobei es sich bei der Bestrahlungsquelle um eine natürliche Bestrahlungsquelle handelt.

**11.** Verfahren nach einem der Ansprüche 1 bis 10, wobei die Bestrahlungsquelle mindestens einen Wellenlängenbereich größer als 280 nm emittiert.

**12.** Verfahren nach Anspruch 11, wobei die Bestrahlungsquelle mindestens einen Wellenlängenbereich zwischen 315 nm und 800 nm emittiert.

**Claims**

**1.** Process for the photocatalytic reduction of carbon dioxide carried out in the liquid phase and/or in the gas phase, said process comprising the following steps:

a) a feedstock containing carbon dioxide and at least one sacrificial compound is brought into contact with a

photocatalyst comprising at least one semiconductor based on microporous crystalline metal sulfide in the form of a solid comprising a chemical composition expressed on an anhydrous basis, in terms of moles, by the following general formula:

$$X_aY_bS_8: cR$$

where

X represents at least one tetravalent element chosen from Sn, Ge, Ti or Zr,
Y represents at least one divalent metal chosen from Zn, Cd or Ni,
R represents at least one nitrogenous organic species,
S is sulfur,
"a" is the molar amount of X of between 0.1 and 5;
"b" is the molar amount of Y of between 0.2 and 8;
"c" is the molar amount of the nitrogenous organic species R of between 0 and 4;

b) the photocatalyst is irradiated by at least one irradiation source producing at least one wavelength which is lower than the bandgap width of said photocatalyst, of between 1.24 and 3 eV, said step b) being carried out at a temperature of between -10°C and 200°C and at a pressure of between 0.01 MPa and 70 MPa.

**2.** Process according to Claim 1, wherein said photocatalyst comprising at least one semiconductor is provided in the form of a solid comprising a chemical composition expressed on an anhydrous basis, in terms of moles, defined by the following general formula:

$$Sn_aZn_bS_8: cR$$

where

R represents at least one nitrogenous organic species;
S is sulfur;
"a" is the molar amount of Sn of between 0.1 and 5;
"b" is the molar amount of Zn of between 0.2 and 8;
"c" is the molar amount of the nitrogenous organic species R of between 0 and 4.

**3.** Process according to either of Claims 1 and 2, wherein "c" is between 0.2 and 4.

**4.** Process according to Claim 3, wherein said solid exhibits an X-ray diffraction diagram including at least the lines listed in the table below:

| 2 theta (°) | $d_{hkl}$ (Å) | $I_{rel}$ |
|---|---|---|
| 7.95 | 11.11 | S |
| 8.88 | 9.95 | m |
| 10.18 | 8.68 | w |
| 11.10 | 7.97 | VS |
| 11.72 | 7.54 | S |
| 15.37 | 5.76 | m |
| 16.71 | 5.30 | w |
| 17.36 | 5.11 | w |
| 21.49 | 4.13 | m |
| 22.30 | 3.98 | m |
| 23.31 | 3.81 | w |
| 30.05 | 2.97 | w |

(suite)

| 2 theta (°) | $d_{hkl}$ (Å) | $I_{rel}$ |
|---|---|---|
| 33.34 | 2.69 | w |
| 35.96 | 2.50 | w |
| 40.80 | 2.21 | w |
| where VS = very strong; S = strong; m = medium; w = weak. | | |

**5.** Process according to any one of Claims 1 to 4, wherein R is an organic compound comprising at least two nitrogen atoms.

**6.** Process according to Claim 5, wherein R is 1,3-bis(4-piperidinyl)propane.

**7.** Process according to any one of Claims 1 to 6, wherein said semiconductor exhibits a micropore volume, determined by nitrogen porosimetry, of between 0.01 and 0.50 $cm^3/g$.

**8.** Process according to any one of Claims 1 to 7, wherein, when said process is carried out in the gas phase, the sacrificial compound is a gaseous compound chosen from water, ammonia, hydrogen, methane and an alcohol.

**9.** Process according to any one of Claims 1 to 7, wherein, when the process is carried out in the liquid phase, the sacrificial compound is a soluble solid or liquid compound chosen from water, ammonia, an alcohol, an aldehyde or an amine.

**10.** Process according to any one of Claims 1 to 9, wherein the irradiation source is a natural irradiation source.

**11.** Process according to any one of Claims 1 to 10, wherein the irradiation source emits at at least one wavelength range greater than 280 nm.

**12.** Process according to Claim 11, wherein the irradiation source emits at at least one wavelength range of between 315 nm and 800 nm.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 2013252798 A
- CN 109331883
- CN 109225273
- CN 109046385
- CN 106006717


### Littérature non-brevet citée dans la description


- **HALMANN et al.** *Solar Energy*, 1983, vol. 31 (4), 429-431
- **ANPO et al.** *J. Phys. Chem. B*, 1997, vol. 101, 2632-2636
- **MORI et al.** *RSC Adv.*, 2012, vol. 2 (8), 3165-3172
- **O. STROYUK et al.** *Chem. Soc. Rev.*, vol. 2108 (47), 5354
- CRC Handbook of Chemistry and Physics. CRC press, 2000
- **F. ROUQUÉROL** ; **J. ROUQUÉROL** ; **K. SING**. Adsorption by powders and porous solids. Principles, methodology and applications. Academic Press, 1999
- **BRUNAUER-EMMETT-TELLER**. *The Journal of American Society"*, 1938, vol. 60, 309